# EUROPEAN PATENT SPECIFICATION

(11) **EP 1 642 612 B1**
(45) Date of publication and mention of the grant of the patent: **12.08.2015**
(21) Application number: 05077193.0
(22) Date of filing: 09.04.1996
(51) Int. Cl.: A61M 25/10

(54) **Balloon catheter with lobated balloon and method for manufacturing such a catheter**
Ballonkatheter mit lobalem Ballon und Verfahren zur Herstellung eines solchen Katheters
Cathéter à ballon avec ballon lobé et méthode de fabrication d'un tel cathéter

(30) Priority: 10.04.1995 NL 1000106
(43) Date of publication of application: 05.04.2006
(62) Divisional of application: 96200982.5
(73) Proprietor: CORDIS EUROPA N.V., NL-9301 LJ Roden (NL)
(72) Inventor: Weber, Jan, 9302 ER Roden (NL); Van Muiden, J.G.M., 9321 AZ Peize (NL)
(74) Representative: D'Halleweyn, Nele Veerle Trees Gertrudis

(56) References cited:
- WO-A-93/19802
- WO-A-94/05365
- WO-A-94/23787
- US-A- 5 108 370
- US-A- 5 308 356

## Description

The invention relates to a catheter comprising in the usual manner a tube-like basic body with a proximal and a distal end and an expandable balloon member arranged close to the distal end to the basic body.

Such balloon catheters, e.g. known from WO 9405365, WO 95/17223, are for instance used for the purpose of dilating narrowed blood vessels. During treatment the balloon is advanced via a blood vessel of the patient to the constriction, where it is expanded by supplying a liquid or gas under pressure from the proximal end to the balloon, as a result of which the blood vessel will be dilated at that site.

With this treatment it is often necessary that the balloon is kept in its expanded state for a certain period of time. In the known catheter grooves are formed in the balloon to prevent that during this time the blood vessel is occluded completely, as a result of which the tissue cells upstream from the expanded balloon would be deprived of nourishment and oxygen. When the dilatation treatment would take up too much time the tissue in question could be damaged.

With the catheter according to the invention, as characterized in claim 1, this effect is further improved. In the expanded state the lobes carry out the dilatation, while in between the lobes channels are formed through which a sufficient amount of blood can flow. The risk that the tissue upstream receives too little nourishment and/or oxygen is consequently removed.

An additional advantage of the stiff bands in between the lobes is that they will display an axial bursting direction when too much pressure is applied to the material of which the balloon has been made. The axial tatters will not damage the blood vessel on removal of the balloon.

The relatively stiff parts of the balloon member are formed by making them of a fibre-reinforced or cross-linked thermoplastic material respectively. The total, unexpanded diameter of the balloon can consequently remain small.

The balloon is of the pre-formed type, the lobes will be able to fold properly, on deflation of the balloon, against the basic body.

The invention also relates to and provides a method for manufacturing a catheter as described above. This method is characterized in claim 4. The basic material which does not expand in the mould cavity, as it is positioned against the inwardly protruding ridges of the lobated mould cavity, retains a relatively greater thickness so that it remains relatively stiff. The intermediate sections which have expanded into the lobes, will obtain a thinner wall and will consequently become relatively pliable.

Preferably the method as set out in claim 5 is employed. The relatively stiff parts can consequently obtain a suitable stiffness.

By employing the measure as set out in claim 6, a balloon member can be manufactured of which the relatively stiff sections and the lobes extend in a helical pattern as a result of which this balloon becomes properly foldable.

The invention is explained in greater detail in the following description with reference to the attached drawings.
- Figure 1: is a simplified view of a balloon catheter according to the invention during treatment.
- Figure 2: is a partly broken away perspective view of a distal end-section of a catheter according to a first embodiment of the invention, in which case the balloon member is unexpanded.
- Figure 3: illustrates the balloon member of figure 2 in expanded state.
- Figure 4: shows a balloon member of the pre-formed type in unexpanded state.
- Figure 5: illustrates schematically the method according to the invention.
- Figure 6: is a partial view of another embodiment of the catheter according to the invention.
- Figure 7: is a partial view of yet another embodiment.

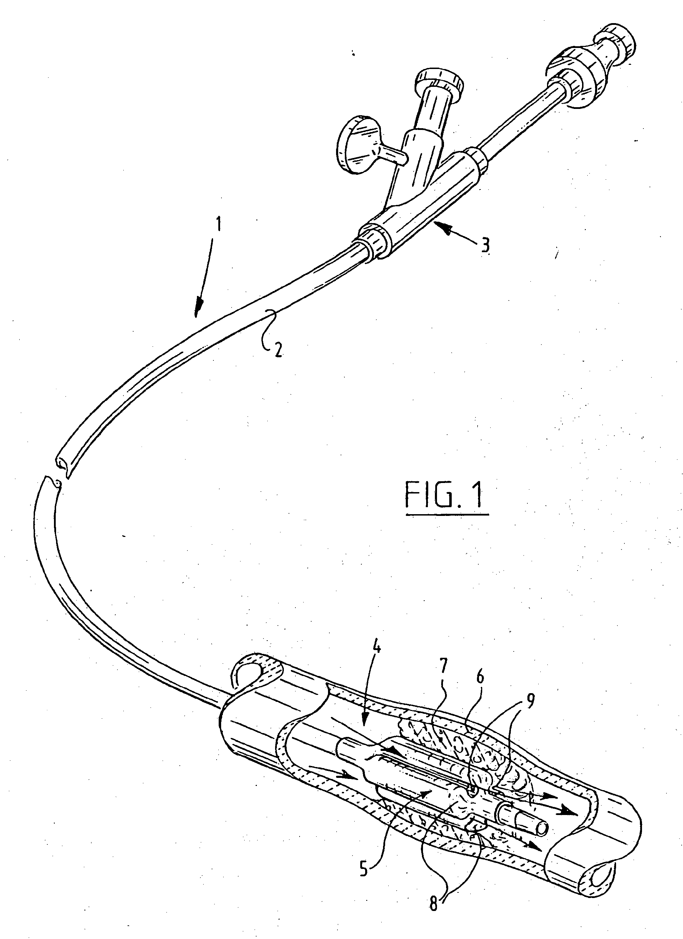

The catheter 1 according to the invention shown in figure 1 comprises a tube-like basic body with a proximal end 3 and a distal end 4. The distal end 4 is provided with a balloon member 5.

The balloon member 5 has in the illustrated expanded state a number of lobes 8 with channels 9 formed in between. In the illustrated position of use, the balloon member 5 of the catheter 1 has been positioned at a constriction formed by deposits 7 inside a blood vessel 6 of a patient. By expanding the balloon member 5 the constriction is treated in a manner known as such. As during the expansion of the balloon member 5 channels 9 are formed in between the lobes 8, flow through the blood vessel 6 will not be blocked completely. As has been indicated with the arrows, blood can still flow through the blood vessel 6, also in the expanded state of the balloon 5, so that tissue located upstream from it is not starved of oxygen and nourishment during treatment.

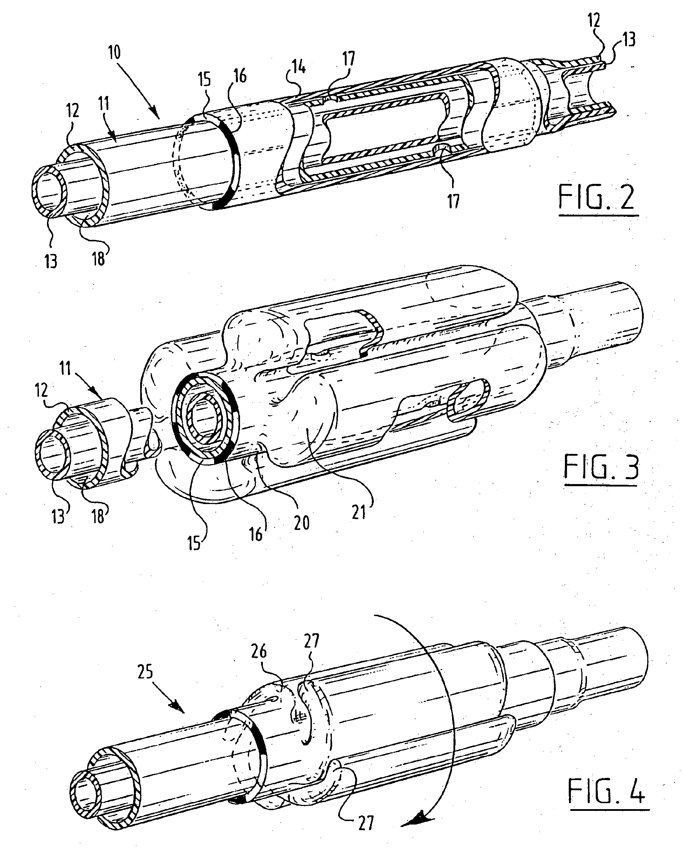

Figure 2 and 3 show in greater detail the distal end of an embodiment of the catheter according to the invention: figure 2 in unexpanded state and figure 3 in expanded state.

The catheter 10 in these figures comprises a basic body 11 which has been assembled form an outer tube-like body and an inner tube-like body 13 received in a lumen 18 thereof. At the distal end the inner tube-like body 13 is connected with the outer tube-like body 12 around its entire circumference. A balloon member 14 has been arranged to the outer tube-like body 12. With both ends it has been connected with the circumference of the tube-like body 12.

As has been indicated in the figures 2 and 3 the balloon member 14 comprises a number of relatively stiff sections 16 extending substantially in the longitudinal direction of the basic body 11 and relatively pliable sections 15 extending in between.

For the purpose of expanding the balloon 14 a liquid or gas under pressure is supplied via the lumen 18 from the proximal end. This medium under pressure flows via the openings 17 in the wall of the outer tube-like body 12 into the balloon member 14. As a result of the supplied pressure, the relatively pliable sections 15 of the balloon member 14 expand into lobes 21 as shown in figure 3. Channels 20 remain in between the lobes 21, as the relatively stiff sections 16 do not expand or expand less.

The relatively stiff sections 16 can be formed because the material of the balloon member 14 has different properties to that of the sections 15. The relatively stiff sections 16 can also be formed by connecting them in a longitudinal direction with the outer tube-like element 12 of the basic body 11, whereas the intermediate, relatively pliable sections 15 will not be connected with the basic body and can consequently expand freely.

The balloon member 14 can have been manufactured of an elastic material, so that the relatively pliable sections 15 stretch on expansion into the lobes 21 shown in figure 3. It is also possible to pre-form the balloon, as a result of which it will obtain the expanded form but can be folded into a smaller diameter. Such an embodiment has been illustrated in figure 4. The catheter 25 has a balloon member 26 with pre-formed lobes 27. By rotating the balloon member 26 in the direction indicated by the arrow, the unexpanded lobes are folded against the basic body, so that at the distal end the catheter will obtain a relatively small cross-section. In this state the balloon member can be introduced into a patient.

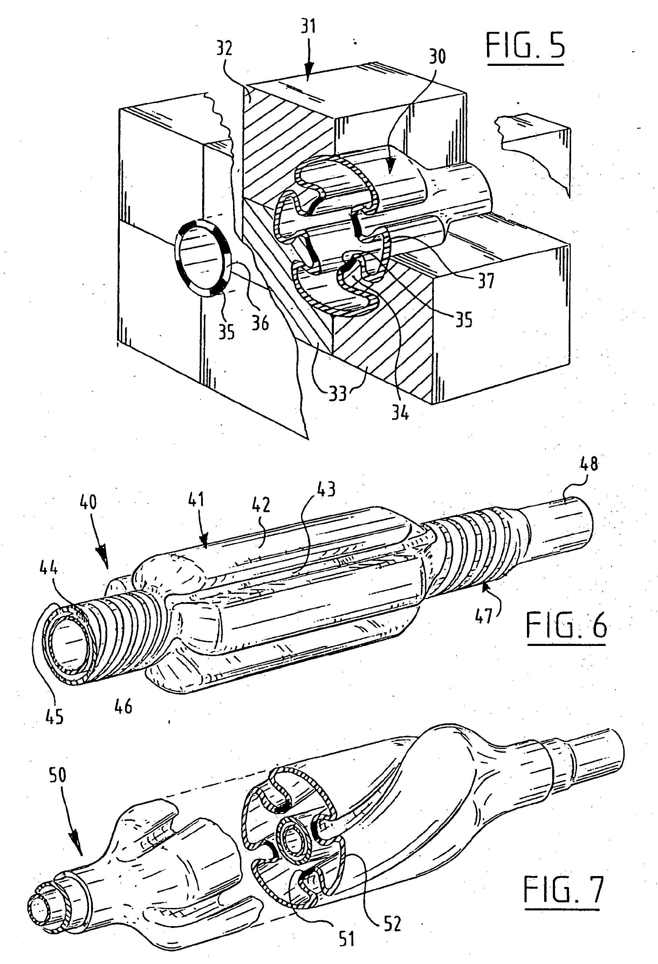

Figure 5 illustrates schematically a method for manufacturing a balloon member for a catheter according to the invention. With the method shown here a balloon member 30 of the pre-formed type is manufactured. For this purpose a mould 31 is used which comprises, in this example of an embodiment, four mould-sections 32,33 movable in relation to one another. In the four mould-sections 32, 33 a mould cavity is defined which has four lobes in cross-section. The lobes are bounded each time on either side by ridges 34 protruding inwardly inside the mould cavity.

For the purpose of forming the balloon 30, a piece of tube-like basic material is placed inside the mould cavity and the mould-sections 31, 32 are placed against each other, so that the mould cavity is defined. Next the material of which the semimanufacture has been made is heated and an overpressure is generated inside it, as a result of which this material will expand and place itself against the wall of the mould cavity. The balloon 30 will thus obtain the shape corresponding to that of the mould cavity. After cooling, the balloon member 30 can be taken from the mould.

According to the illustrated preferred embodiment, the semimanufacture has been formed with, in cross-section, alternating sections 35, 36 made of relatively stiff and pliable material respectively. The semimanufacture has been received in the mould 31 in such a way that the sections 35 containing relatively stiff material are positioned against the ridges 34. As a result only the relatively pliable sections 36 expand during moulding. The relatively stiff sections 35 form so to speak stiffening ribs in the material of the balloon 30. The lobes 37 are connected each time on both sides with such a stiffening rib.

With the embodiment of the catheter 40 according to figure 6, the semimanufacture of which the balloon 41 has been manufactured has been obtained by strip-shaped co-extrusion of relatively stiff material 44 and relatively pliable material 45. It is seen to that a good bond is obtained between the relatively stiff material and the relatively pliable material during co-extrusion. In a first section of the balloon 41, with which it is connected to the basic body, the strips 44, 45 have been extruded in a helical pattern by using a rotating extrusion head. In the expandable section of the balloon 41 the coextruded bands of material 44, 45 extend axially as during the manufacturing process of the semimanufacture the rotating mould head has been stopped. Thus lobes 42 extending axially can be formed with bounded channels 43 in between. The end-section 47 first comprises a section in which the bands of material extend in a helical pattern and finally an end-section 48 comprising only the relatively pliable material 45. The supply of the relatively stiff material 44 has been switched off during the extrusion of this end-section 48.

With the catheter 50 of figure 7 the relatively stiff sections 51 extend in a helical pattern with a large pitch. Consequently the lobes 52 also extend in such a helical pattern. In the unexpanded state of this balloon, its lobes 52 can fold together into a small diameter, in particular by twisting the balloon member in the unexpanded state around its longitudinal axis. The lobes 52 are then folded closely against the basic body.

With an embodiment not shown here in greater detail, the relatively stiff sections of the balloon can be made of a thermoplastic material containing fibrous reinforcement or by using material which can be cross-linked. If the latter is the case the balloon will be irradiated with for instance ultraviolet light or an electron beam. Consequently transverse connections are formed between the molecules as a result of which the relatively stiff sections of the balloon are formed. These types of material have a high tensile strength following irradiation and provide a high degree of resistance against deformation when the balloon is being inflated.

The term "material" used in this application concerns both homogenous and non-homogenous materials. The non-homogenous materials include for instance materials containing fibrous reinforcement or materials to which additives have been added which form transverse connections or cross-links between the molecules as a result of irradiation with for instance ultraviolet light or an electron beam in order to provide this material with a greater stiffness and tensile strength.

## Claims

1. Catheter comprising a tube-like basic body with a proximal and a distal end and an expendable balloon member arranged close to the distal end of the basic body, the tube-like material of the balloon member comprising a number of relatively stiff sections extending on a small diameter in a longitudinal direction of the basic body and relatively pliable sections that are pliable relative to the stiff sections extending in between, wherein in an expanded form of the balloon member the relatively stiff sections remain substantially at the same diameter as in a non-expanded form and the relatively pliable sections form lobes,
**characterized in that**
the balloon member is pre-formed that the relatively stiff sections are made of a fibre-reinforced or cross-linked thermoplastic material and that the number of relatively stiff sections of the tube-like material are integrally formed in said tube-like material and alternate with the sections of relatively pliable material, said relatively stiff sections being formed by material with a greater specific stiffness than the material of the relatively pliable sections.

2. Catheter as claimed in claim 1, wherein the relatively stiff sections are formed by material containing additives which have formed cross connections in the material at molecular level by irradiation with for instance ultraviolet light or an electron beam.

3. Catheter as claimed in claim 1, wherein the relatively stiff sections extend in a helical pattern.

4. Method for manufacturing a catheter according to one of the previous claims, comprising the providing of a piece of tube-like basic material, the manufacturing of a balloon member by blow moulding inside a mould and the arranging of the balloon member to the basic body, wherein the manufacturing of the balloon member comprises the providing of a mould with a mould cavity of which the cross-section is lobated, the receiving inside the mould of a tube-like semimanufacture, the tube-like material being formed with, in cross-section, alternating sections of relatively stiff and relatively pliable material, positioning the semimanufacture with the sections of relatively stiff material against inwardly protruding ridges of the lobated mould cavity, and the deformation of the semimanufacture by blow moulding until it corresponds to the shape of the mould cavity.

5. Method as claimed in claim 4, wherein the semimanufacture is manufactured by strip-like co-extrusion of relatively stiff and relatively pliable material.

6. Method as claimed in claim 5, wherein the semimanufacture is rotated in relation to an extrusion head during extrusion, so that the bands of material which are formed extend in a helical pattern.

## Patentansprüche

1. Katheter, umfassend einen schlauchartigen Grundkörper mit einem proximalen und einem distalen Ende und einem dehnbaren Ballonglied, welches nahe des distalen Endes des Grundkörpers angeordnet ist,
wobei das schlauchartige Material des Ballongliedes eine Anzahl von relativ steifen Abschnitten, die sich auf einem kleinen Durchmesser in einer Längsrichtung des Grundkörpers erstrecken, und relativ formbare Abschnitte umfasst, die relativ zu den dazwischen sich erstreckenden steifen Abschnitten formbar sind,
wobei die relativ steifen Abschnitte in einer gedehnten Form des Ballongliedes im Wesentlichen bei demselben Durchmesser bleiben wie in einer nicht gedehnten Form und die relativ formbaren Abschnitte Lappen bilden,
dadurch gekenzeichnet, dass das Ballonglied vorgeformt ist, dass die relativ steifen Abschnitte aus einem faserverstärkten oder vernetzten thermoplastischen Material gebildet sind
und dass die Anzahl der relativ steifen Abschnitte des schlauchartigen Materials in dem schlauchartigen Material integral gebildet sind und sich mit den Abschnitten aus relativ formbarem Material abwechseln,
wobei die relativ steifen Abschnitte aus einem Material mit einer größeren spezifischen Steifheit gebildet sind als das Material der relativ formbaren Abschnitte.

2. Katheter nach Anspruch 1, wobei die relativ steifen Abschnitte aus einem Material gebildet sind, welches Zusätze enthält, die in dem Material in molekularer Ebene durch Bestrahlung mit beispielsweise ultraviolettem Licht oder einem Elektronenstrahl gebildete Vernetzungen aufweisen.

3. Katheter nach Anspruch 1, wobei die relativ steifen Abschnitte sich in einem helikalen Muster erstrecken.

4. Verfahren zum Herstellen eines Katheters nach einem der vorhergehenden Ansprüche, umfassend das Bereitstellen
eines Stückes aus schlauchartigem Material, das Herstellen eines Ballongliedes durch Blasformen innerhalb einer Form und das Herrichten des Ballongliedes zu dem Grundkörper, wobei das Herstellen des Ballongliedes umfasst
das Bereitstellen einer Form mit einem Formhohlraum, dessen Querschnitt lappenförmig ist,
das Aufnehmen eines schlauchartigen Halberzeugnisses innerhalb der Form, wobei das schlauchartige Material mit im Querschnitt abwechselnden Abschnitten aus relativ steifem und relativ formbarem Material gebildet ist,
das Positionieren des Halberzeugnisses mit den Abschnitten aus relativ steifem Material gegen nach innen vorstehende Rippen des Lappen aufweisenden Formhohlraums
und das Verformen des Halberzeugnisses durch Blasformen, bis es der Form des Formhohlraums entspricht.

5. Verfahren nach Anspruch 4, wobei das Halberzeugnis durch streifenartiges Co-Extrudieren des relativ steifen und relativ formbaren Materials hergestellt wird.

6. Verfahren nach Anspruch 5, wobei das Halberzeugnis während des Extrudierens in Bezug auf den einen Extrusionskopf gedreht wird, derart, dass die gebildeten Materialbänder sich in einem helikalen Muster erstrecken.

## Revendications

1. Cathéter comprenant un corps basique de type tubulaire avec une extrémité proximale et une extrémité distale et un élément à ballonnet dilatable agencé près de l'extrémité distale du corps basique, le matériau de type tubulaire de l'élément à ballonnet comprenant un certain nombre de sections relativement rigides s'étendant sur un petit diamètre dans une direction longitudinale du corps basique et des sections relativement pliables qui sont pliables par rapport aux sections rigides s'étendant entre elles, dans lequel, sous une forme dilatée de l'élément de ballonnet, les sections relativement rigides restent sensiblement au même diamètre que sous une forme non dilatée et les sections relativement pliables forment des lobes,
**caractérisé en ce que** l'élément à ballonnet est préformé, que les sections relativement rigides sont faites d'un matériau thermoplastique renforcé par des fibres ou réticulé, et que toutes les sections relativement rigides du matériau de type tubulaire sont formées d'une seule pièce dans ledit matériau de type tubulaire et alternent avec les sections de matériau relativement pliable, lesdites sections relativement rigides étant formées en un matériau ayant une rigidité spécifique supérieure au matériau des sections relativement pliables.

2. Cathéter selon la revendication 1, dans lequel les sections relativement rigides sont formées en un matériau contenant des additifs qui forment des connexions croisées dans le matériau au niveau moléculaire par irradiation avec, par exemple, une lumière ultraviolette ou un faisceau d'électrons.

3. Cathéter selon la revendication 1, dans lequel les sections relativement rigides s'étendent selon un motif hélicoïdal.

4. Procédé de fabrication d'un cathéter selon l'une des revendications précédentes, comprenant la fourniture d'un morceau de matériau basique de type tubulaire, la fabrication d'un élément à ballonnet par moulage en soufflant à l'intérieur d'un moule et l'agencement de l'élément à ballonnet sur le corps basique, dans lequel la fabrication de l'élément à ballonnet comprend la fourniture d'un moule avec une cavité de moule dont la section transversale est lobée, la réception à l'intérieur dudit moule d'un produit semi-ouvré de type tubulaire, le matériau de type tubulaire étant formé avec, de manière transversale, des sections alternées de matériau relativement rigide et de matériau relativement pliable, le positionnement du produit semi-ouvré avec les sections de matériau relativement rigide contre des arêtes en saillie vers l'intérieur de la cavité de moule lobée, et la déformation du produit semi-ouvré par moulage par soufflage jusqu'à ce qu'il corresponde à la forme de la cavité de moule.

5. Procédé selon la revendication 4, dans lequel le produit semi-ouvré est fabriqué par co-extrusion de type en bande d'un matériau relativement rigide et relativement pliable.

6. Procédé selon la revendication 5, dans lequel le produit semi-ouvré est mis en rotation relativement à une tête d'extrusion pendant l'extrusion, de sorte que les bandes de matériau qui sont formées s'étendent selon un motif hélicoïdal.
